# EUROPEAN PATENT SPECIFICATION

(11) **EP 3 054 885 B1**
(45) Date of publication and mention of the grant of the patent: **29.04.2020**
(21) Application number: 14792885.7
(22) Date of filing: 26.09.2014
(51) Int. Cl.: A61B 34/20, A61B 8/00, A61B 8/08

(54) **IMAGE GUIDANCE SYSTEM WITH USER DEFINABLE REGIONS OF INTEREST**
BILDFÜHRUNGSSYSTEM MIT ANWENDERDEFINIERBAREN INTERESSEBEREICHEN
SYSTÈME DE GUIDAGE D'IMAGES À RÉGIONS D'INTÉRÊT DÉFINISSABLES PAR L'UTILISATEUR

(30) Priority: 30.09.2013 US 201361884197 P
(43) Date of publication of application: 17.08.2016
(73) Proprietor: Koninklijke Philips N.V., 5656 AG Eindhoven (NL)
(72) Inventor: DALAL, Sandeep M., NL-5656 AE Eindhoven (NL); PARTHASARATHY, Vijay, NL-5656 AE Eindhoven (NL)
(74) Representative: Philips Intellectual Property & Standards
(86) International application number: PCT/IB2014/064852
(87) International publication number: WO 2015/044901

(56) References cited:
- WO-A1-2010/073165
- WO-A2-2012/143885
- US-A1- 2008 200 808
- US-A1- 2012 289 820

## Description

### BACKGROUND:

### Technical Field

This disclosure relates to medical instruments and more particularly to bidirectional data transfer and visualization of real-time interventional information in an ultrasound system.

### Description of the Related Art

Ultrasound-guided image interventions allow clinicians to view a patient's anatomy and interventional devices inserted into the tissue in real time. Image-guided interventional procedures using three-dimensional ultrasound can range from interventions in cardiology, oncology, radiology, etc. In these interventional procedures, two-dimensional or three-dimensional ultrasound images are visualized on the screen of the ultrasound system to guide the clinician in accurately placing interventional devices at target locations in the patient's anatomy and making intra-procedural real time measurements with sensors embedded on devices, such as catheters, guidewires, etc. While these images can be captured by the ultrasound system and displayed on the ultrasound system screen, the measurements are usually displayed on separate consoles provided by the device manufacturers.

U.S. Patent Publication No. 2008/200808; WO Patent Publication No. 2012/143885; and WO Patent Publication No. 2010/073165 disclose imaging systems for interventional procedures.

### SUMMARY

In accordance with the present principles, an image guidance system is defined by claim 1. A method for image guidance is defined by claim 14. Advantageous embodiments are provided in the dependent claims.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing an image guidance system which employs a bidirectional communication channel to couple an imaging system with computation engine, in accordance with one illustrative embodiment;
FIG. 2 is a display of an imaging system showing a critical structure selected by a user, in accordance with one illustrative embodiment;
FIG. 3 shows ultrasound images in a multi-planar format, in accordance with one illustrative embodiment;
FIG. 4 is a display of an imaging system showing real time sensor data, in accordance with one illustrative embodiment;
FIG. 5 shows a spatial bounding box constructed around a region of interest, in accordance with one illustrative embodiment; and
FIG. 6 is a block/flow diagram showing a method for image guidance, in accordance with one illustrative embodiment.

### DETAILED DESCRIPTION OF EMBODIMENTS

In accordance with the present principles, systems, workstations and methods are provided for image guided interventions. An ultrasound system may be employed to generate three-dimensional ultrasound image streaming in the context of image-guided interventional procedures using a medical device, e.g., needle, catheter, guidewire etc. The interventional procedure may also employ one or more sensors measuring attributes of the tissue, such as, e.g., temperature, contact force, etc. A computation engine registers the imaging coordinate system of the images and the tracking coordinate system of the tracked medical device and tracked sensors into a global coordinate system. The computation engine may construct one or more two-dimensional (2D) planes. Each plane includes an intersection of at least two of: the tracked medical device, sensors, areas of interest (e.g., critical structures), etc. Multiple planes may be generated for each intersection.

The ultrasound system may be coupled to a computation engine through a bidirectional communication channel. The bidirectional communication channel permits communication of the images and user selected points and areas of interest from the ultrasound system to the computation engine. The bidirectional communication channel also permits communication of the 2D planes from the computation engine to the ultrasound system, as well as any sensor data. The display of the ultrasound system may visualize imaging data in any of the 2D planes as specified by the data communicated from the computation engine. The sensor data may also be displayed on a display of the ultrasound system.

Advantageously, the bidirectional communication channel permits the transmission of data relating to the intervention back into the ultrasound system. In addition, the bidirectional communication channel allows other sources of information, such as, e.g., real time positions of one or more medical devices, distances between real-time positions of medical devices and one or more sensors, distances between real-time positions of interventional devices and user-specified areas of interest (e.g., critical structures in the imaging data), and real-time measurement data from sensors to be displayed back on the screen of the ultrasound system.

It should be understood that the present invention will be described in terms of medical instruments; however, the teachings of the present invention are much broader and are applicable to any fiber optic instruments. In some embodiments, the present principles are employed in tracking or analyzing complex biological or mechanical systems. In particular, the present principles are applicable to imaging procedures of biological systems, procedures in all areas of the body such as the lungs, liver, kidney, abdominal region, gastro-intestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray™ and DVD.

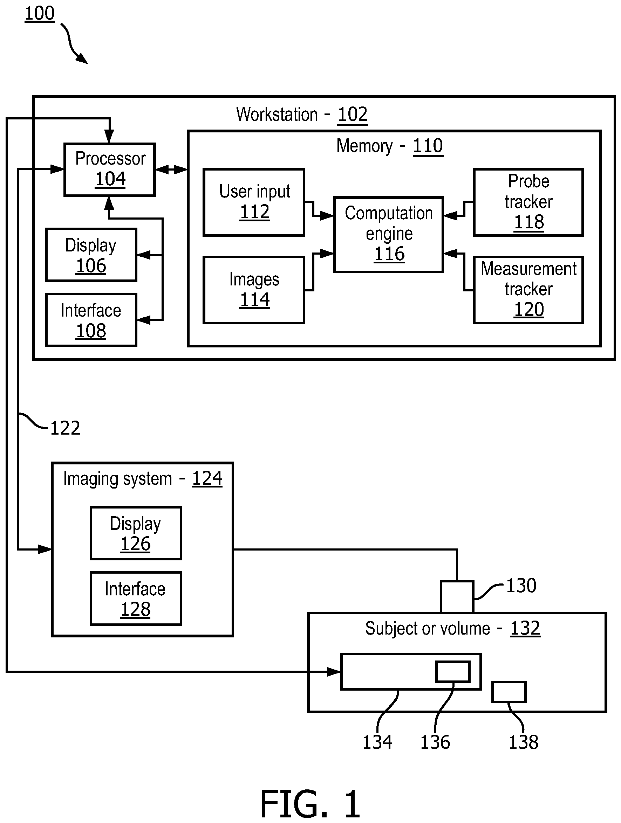

Referring now to the drawings in which like numerals represent the same or similar elements and initially to FIG. 1, a system 100 for spatial tracking and sensing data integration is illustratively shown in accordance with one embodiment. The system 100 may include a workstation or console 102 from which a procedure is supervised and/or managed. The workstation 102 preferably includes one or more processors 104 and memory 110 for storing programs, applications and other data.

The workstation 102 includes a display 106 for viewing, e.g., images or data relating to the procedure. The display 106 may also permit a user to interact with the workstation 102 and its components and functions, or any other element within the system 100. This is further facilitated by an interface 108 which may include a keyboard, mouse, a joystick, a haptic device, or any other peripheral or control to permit user feedback from and interaction with the workstation 102. It should be understood that the components and functions of the system 100 may be integrated into one or more systems or workstations, or may be part of a larger system or workstation.

The imaging system 124 preferably includes an ultrasound (US) system having a tracked probe or transducer 130 to provide a live stream of two-dimensional (2D) or three-dimensional (3D) volumetric images 114. It should be understood that imaging system 124 is not limited to an ultrasound system, but rather may include any imaging system, particularly those suitable for real time imaging, such as, e.g., fluoroscopy, etc. The probe 130 is preferably tracked using a tracking device (not shown), such as, e.g., an electromagnetic (EM) tracking device, optical tracking device, etc. The tracking device allows real time spatial tracking of the probe 130 in an imaging tracking system. The imaging tracking system also enables the real time tracking of the position and pose (i.e., orientation) of the medical device 134 in the imaging coordinate system. Spatial tracking information of the probe 130 is stored as probe tracker 118.

The imaging system 124 includes its own display 128 and interface 128 for user interactions. For example, the interface 128 may allow a user to select areas or points of interest on the images as user input 112. The points of interest may include locations of sensors 138, critical structures within the subject 132, etc. as visualized in the images 114. The user selected points are selected in the imaging tracking system and transmitted to workstation 102. In one exemplary embodiment, the display 128 may show 3D images in a multi-planar format, i.e., multiple planes (typically 2, but may include more). The 3D images may be sliced along arbitrary, user selected planes. Advantageously, the imaging system 124 is coupled to the workstation 102 via bidirectional communication channel 122, which is capable of conveying 3D images and metadata (e.g., image related attributes, user selected point coordinates, etc.) to the workstation 102 as images 114 and capable of receiving data in real time from the workstation 102.

Critical structures are sites or regions in the subject 132 (e.g., tissue) that would be adversely affected by the intersection of either the medical device 132's path or by the execution of the desired therapy with the medical device 132 as placed in the desired position. Critical structures may include, e.g., landmarks such as tumor target sites, blood vessel bifurcation points that are useful for the user to determine poisoning information, or other sites of interest. These locations are selected by the user (using interface 128) in the imaging coordinate system and communicated to the computation engine 116 for conversion into the global coordinate system. In some embodiments, the computation engine 116 constructs 2D planes representing the location of a tracked medical device 124 in real time in proximity to critical structures. Each plane includes, e.g., an intersection of the tip of the device 134 and the critical structures identified by the user. The 2D planes may be sent to the imaging system 124 over the bidirectional communication cable 122. This allows the user to make a visual judgment of the suitability of the tracked device trajectory, closeness to targets or reference points like vessel bifurcations, or the degree to which the device 134 is maintained away from the critical structures.

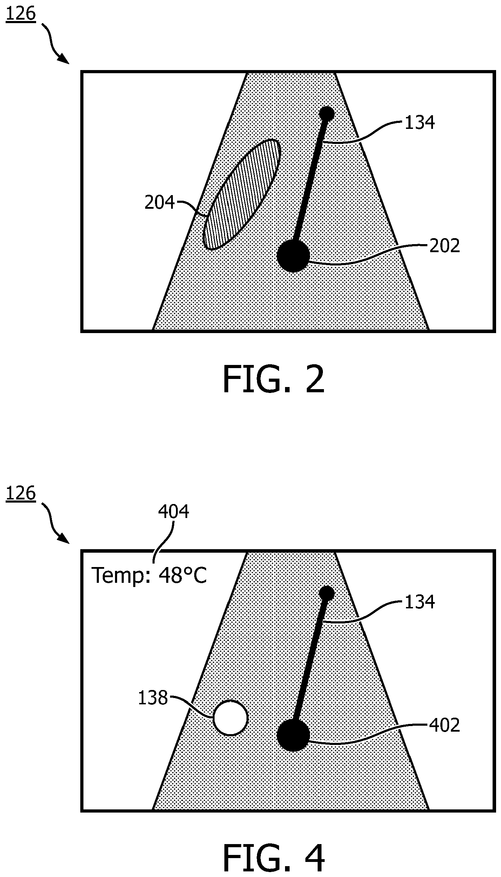

Referring for a moment to FIG. 2, with continued reference to FIG. 1, an exemplary display 126 of the imaging system 124 is shown in accordance with one illustrative embodiment. The display 126 shows a medical device 134 having a tip portion 202. The display 126 may be used for image guidance of an interventional procedure. A critical structure 204 is selected by a user in the imaging coordinate system. The coordinate information is sent to the computation engine 116, which converts the coordinate information into the global coordinate system to construct at least one 2D plane showing the intersection of the selected critical structure 204 and tip portion 202. The 2D plane may also show the intersection between other devices, sensors, areas of interest, etc.

Where the user has selected critical structures (e.g., targets, reference points, etc.) or sensors 138, the display 126 of the imaging system 124 may provide real time distances between the medical device 134 (e.g., tip) and the selected critical structures. The distance may be either from the critical structures to the real time tip location of the medical device 134, or from the critical structure to the closest point of the device 134. For example, this allows the user to steer the medical device 134 safely away from critical structures by ensuring some minimal distance is maintained.

In some embodiments, the user may be notified if the medical device 134 comes within a predetermined threshold distance to a critical structure. In other embodiments, the user may be notified based on an analysis of the sensor data (e.g., temperature exceeds a user defined threshold, contact force is too small or too large, pressure/flow readings are normal/abnormal, etc.). The notification may include, e.g., an audible alarm, a colored light, a flashing light, a pop up message on a display, a haptic response, etc.

Referring back to FIG. 1, the computation engine 116 integrates data from probe tracker 118 and measurement tracker 120 with user input 112 and images 114 from the imaging system 124. The imaging system 124 is coupled to workstation 102 by bidirectional communication channel 122. The bidirectional communication channel 122 permits external devices to send and receive data relating to the intervention back into the imaging system 124. Additionally, the bidirectional channel 122 allows other sources of information, such as, e.g., real time position and measurement data from interventional devices, to be displayed back on the display 126 of the imaging system 124.

The imaging system 124 may provide image guidance for interventional procedures involving one or more objects visible in the images 114. The one or more objects may include devices or instruments 134, one or more sensors 138, etc. The device 134 preferably includes a medical device, such as, e.g., a needle, a catheter, a guidewire, a probe, an endoscope, a robot, an electrode, a filter device, a balloon device, or other medical component, etc. The medical device 134 is coupled to the workstation 102 through cabling, which may include fiber optics, electrical connections, other instrumentation, etc. as needed.

The medical device 134 may be tracked using tracking device 136 coupled to the medical device 134. The tracking device 136 may include, e.g., an EM tracking device, optical tracking device, etc. The tracking device 136 enables real time spatial tracking of the medical device 134 when placed into, e.g., the tissue of subject 132 in the spatial tracking system based on the tracking coordinate system. Spatial tracking information of the medical device 134 is stored in measurement tracker 120.

In some embodiments, there may be multiple medical devices 134 inserted into the subject 132. For example, radiofrequency, cryoablation or microwave ablation proves may be simultaneously present in the tissue of interest. Signals from one or more such devices may provide real time spatial tracking data to the measurement tracker 120.

One or more sensors 138 may be placed within the subject 132. The sensors 138 may be directly coupled to the tracked medical device 134 or independently placed in the vicinity of the target area (e.g., tissue being treated) of the subject 132. The sensors 138 are capable of measuring an attribute of the subject 132 (e.g., tissue of the subject 132) that may be used for treatment monitoring. The sensors 138 may include, e.g., a temperature sensor, a contact force sensor, flow sensor, etc. The sensors 138 may be capable of sending measurement data in real time to workstation 102 through a wired or wireless interface. If the sensor 138 is coupled to the tracked medical device 134, sensor data associated with the measurement and spatial tracking information of the medical device 134 may be transmitted to the workstation 102 simultaneously. Sensor data from sensors 138 are also stored in measurement tracker 120.

Sensor data from sensors 138 may be pre-processed by the computation engine 116 to produce a meaningful output for the user. For example, a temperature sensor data may be directly meaningful, whereas an optical spectrum sensor maps a response per wavelength; however, the meaningful output for the user is not the actual raw data of the spectrum but the signal processing of this response to make a tissue classification determination (e.g. tissue classified as healthy, diseased, treated, or untreated). The nature of the pre-processing may depend on the sensor type. The meaningful output is what is sent back to the imaging system 124.

If there are multiple sensors 138 in the vicinity of the tracked medical device 134, the sensors 138 need to be distinguishable by the user in the images 114 and the identity of each sensor 138 has to be uniquely determined in spatial position. The association of sensors identified in the 3D images 114 and the corresponding data measurement stream is known to the computation engine 116. The computation engine 116 ensures that data for a sensor is presented in concordance with the 2D plane that includes that sensor.

The sensors 138 may or may not be tracked. Non-tracked sensors 138 may be selected by the user (e.g., using interface 128) to identify the sensor locations in the imaging data (in the imaging coordinate system). Tracked sensors 138 may involve, e.g., an EM tracking device, optical tracking device, etc. coupled to the sensors 138. The position of the sensors 138 are tracked in the tracking coordinate system of the tracking device and are reported in real time to the measurement tracker 120. In this manner, a user does not have to identify the sensor 138 in the images 112 and communicate its coordinates to the computation engine 116. Tracked sensors 138 synchronously communicates sensor measurement data with the spatial tracking data to the as measurement tracker 120.

The computation engine 116 receives the following signals. 1) 3D images from the imaging system 124, having a coordinate system (i.e., imaging coordinate system) relative to an arbitrary but fixed point on the probe 130 (e.g., center of the surface of the probe 130). 2) Spatial locations of user selected (user identified) sensors 138 in the imaging coordinate system. 3) Real time spatial tracking information from medical device 134, which are in the coordinate frame of the tracking system 136 (i.e., tracking coordinate system). 4) Real time data measurements from sensors 138. 5) Optionally, real time spatial tracking information from other tracked devices or sensors 138 in or around the target area where the 3D images 114 and medical device 134 are located in the tracking coordinate system.

The computation engine 116 combines the images 114 and real time spatially tracked data into a single global coordinate system using spatial registration techniques. The spatial registration combines the imaging coordinate system (from, e.g., imaging data, selected critical structures, selected sensors, etc.) and tracking coordinate system (from, e.g., tracked medical device 134, tracked sensors 138, etc.) into the global coordinate system. For example, images 114 of the imaging coordinate system may be mapped to the tracking coordinate system using a calibration transform (typically a 4x4 matrix), or vice versa. In another example, the location of the sensor 138 in the imaging coordinate system may be mapped to the tracking coordinate system. Other approaches to registration are also contemplated.

Once all images and device/sensor locations are defined in the global coordinate system, the computation engine 116 defines poses (i.e., positions and orientations) of the device 134 and sensors 138 in 2D planes from the 3D images. The computation engine 116 may use the tip location of the device 134, its orientation, and one or more locations of the sensors 138 to define the poses. For example, a 2D plane can be constructed that shows the entire axis of the device 134 along with its tip in the 3D image. However, there is still one degree of freedom to define in this plane to allow a complete rotation around the axis. The plane can be locked to include a rotation angle around the axis of device 134 that also allows one sensor 138 position to be in-plane. This ensures the tip and shaft of device 134 and one sensor 138 are in one 2D plane. The pose of this plane may be updated with real time spatial tracked position of the device 134 and sensor 138. The computation engine 116 may construct additional planes showing a coplanar intersection between additional devices, sensors, areas of interest, etc. In some embodiments, the computation engine 116 represents the tracked device 134 and/or sensors 138 as virtual objects fused shown on display 126 with or overlaid on the live imaging data, since these instruments and sensors may not be clearly visible in the imaging data itself.

The computation engine 116 uses the bidirectional communication channel 122 to communicate the 3D poses of the 2D planes to the imaging system 124 using the imaging coordinate system. The bidirectional communication channel 122 may be wireless, wired, or a part of a larger cable. If the poses were calculated in the tracking coordinate system, the appropriate conversion may be applied to the pose. The imaging system 124 receives the poses of the 2D planes and calculates the corresponding 2D image from the 3D images as intersected by the 2D plane. The one or more 2D images corresponding to the poses of the 2D planes are displayed on display 126 of the imaging system 124.

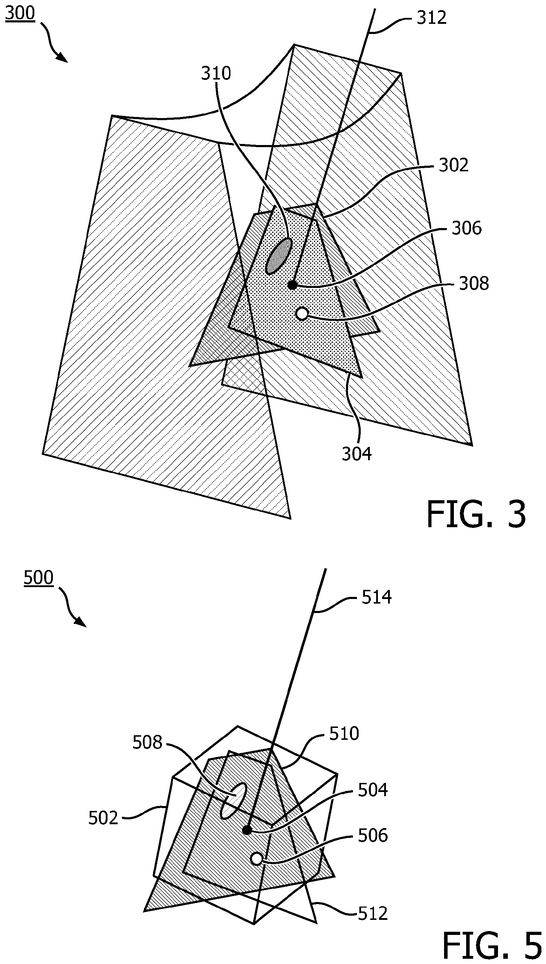

Referring for a moment to FIG. 3, with continued reference to FIG. 1, ultrasound images 300 are shown in a multi-planar format, in accordance with one illustrative embodiment. Two 2D ultrasound images are simultaneously displayed on an ultrasound display, such as the display 126. The ultrasound image includes a first 2D plane 302 and a second 2D plane 304. A medical device 312 having a tip portion 306 may be positioned within a subject (e.g., patient). A sensor 308 and critical structure 310 are located within the vicinity of the target area. The sensor 308 and critical structure 310 may be selected by a user such that the two planes 302, 304 include a selection of the tip portion 306 and shaft of the device 304 with either a user selected sensor 308 or a critical structure 310.

Referring back to FIG. 1, the bidirectional communication channel 122 is used to transmit real time (pre-processed) measurement data from sensors 138 to the imaging system 124. The imaging system 124 may display the corresponding real time sensor measurement data in a graphical user interface appropriate to the measurement type being shown. This may involve display 126 and/or interface 128. The measurement data may be displayed according to a selection of a sensor by the user or automatically depending on which sensors are relevant. Relevant sensors may be determined based on which user-identified critical structures or other structures of interest are present in the visualization at any moment.

Referring for a moment to FIG. 4, with continued reference to FIG. 1, an exemplary display 126 of the imaging system 124 is shown in accordance with one illustrative embodiment. The display 126 shows a medical device 134 having a tip portion 402. A sensor 138 is selected by a user such that at least one 2D plane includes the sensor 138 with the tip portion 402. The sensor's data can be communicated in real time and visualized on the display 126 as real time sensor value 404.

Referring back to FIG. 1, in some embodiments, a spatial bounding box may be constructed. A spatial bounding box is a 3D volume containing a region of interest to the user. Typically, this region of interest includes the tip of device 134, a portion of the shaft of the device 134 close to the tip, and one or more sensors 138 all within the bounding box. The coordinates of the spatial bounding box may be transmitted to the imaging system 124 through bidirectional communication channel 122.

The imaging system 124 receives coordinates of the spatial bounding box and determines a 3D sub-volume of the 3D ultrasound image. This allows the ultrasound system to optimally render only the sub-volume view and 2D planes within the sub-volume. The signal processing attributes, such as, e.g., gain, focus, depth, time-gain-compensation (TGC), frame rate, visualization enhancement, etc., of the display 126 of the imaging system 124 can be dynamically optimized according to the sub-volume locations. Higher frame rates of 3D acquisition may be possible when the 3D region of interest is known to the imaging system.

Referring for a moment to FIG. 5, with continued reference to FIG. 1, areas of interest within a spatial bounding box 500 is shown in accordance with one illustrative embodiment. The spatial bounding box 502 is constructed around a region on interest. The region of interest includes a tip portion 504 of a medical device 514, critical structure 508 and sensor 506 locations. The bounding box coordinates are communicated to the imaging system using the bidirectional communication channel 122 allowing the imaging system to optimize the view within the bounding box. The bounding box 502 includes a first 2D plane 510 and a second 2D plane 512. The sensor 506 and critical structure 508 may be selected by a user such that the two planes 510, 512 include a selection of the tip portion 504 with either a user selected sensor 506 or a critical structure 508.

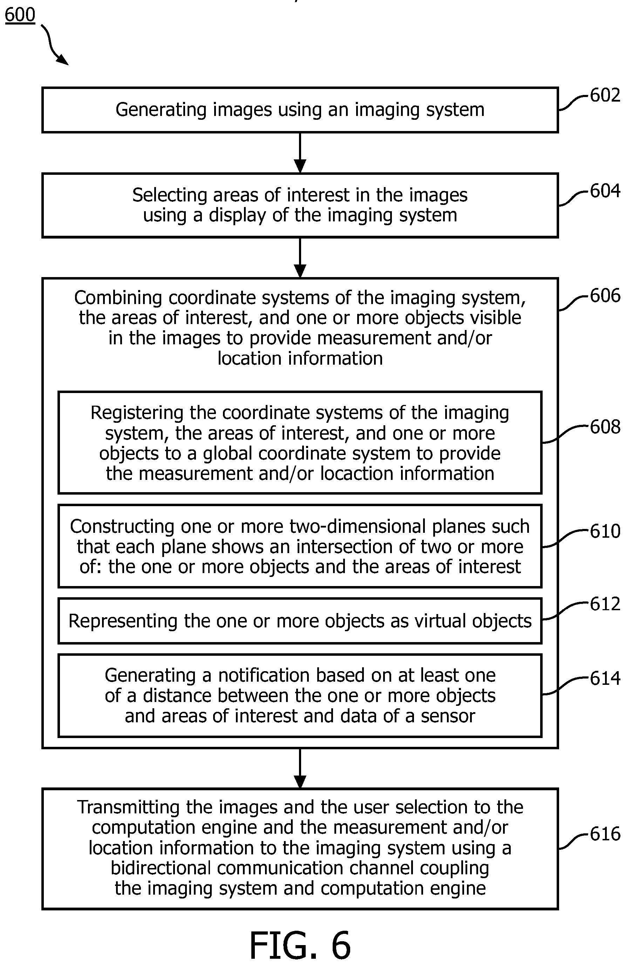

Referring now to FIG. 6, a block/flow diagram showing a method for image guidance 600 is illustratively depicted in accordance with one embodiment. In block 602, images (e.g., 3D) of a subject (e.g., patient, volume, etc.) are generated using an imaging system. The imaging system preferably includes an ultrasound system having a tracked probe. In block 604, areas of interest in the images are selected using a display of the imaging system. Areas of interest may include (non-tracked) sensors, critical structures, etc.

In block 606, coordinate systems of the imaging system, the areas of interest, and one or more objects visible in the images are combined to provide measurement and/or location information. The one or more objects may include one or more devices (e.g., medical instruments), one or more sensors, etc. Measurement and/or location information may include visualization information, data from a sensor, etc. The imaging data and areas of interest identified in the imaging data as selected by the user (e.g., non-tracked sensors, critical structures) are tracked in an imaging coordinate system. The spatial locations of the tracked device and tracked sensors are tracked in a tracking coordinate system. In block 608, combining includes registering the coordinate systems of the imaging system, the areas of interest, and the one or more objects to a global coordinate system to provide the measurement and/or location information.

In block 610, one or more 2D planes are constructed such that each 2D plane shows an intersection of at least two or more of: the one or more objects (e.g., devices, sensors, etc.) and the areas of interest (e.g., critical structures, points of interest, etc.). In some embodiments, a spatial bounding box is constructed for a target area in the 2D planes. The target area may include the tracked device, one or more sensors, areas of interest, etc. In block 612, the one or more objects may be represented as virtual objects in the display of the imaging system. In block 614, a notification is generated. The notification may be based on a distance between the one or more objects and areas of interest or based on the data of the one or more sensors.

In block 616, the 3D images and the user selection are transmitted to the computation engine and the measurement and/or location information are transmitted to the imaging system using a bidirectional communication channel. The bidirectional communication channel couples the imaging system and the computation engine, allowing sources of information to be displayed on the imaging system.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for bidirectional data transfer and visualization of real-time interventional information in an ultrasound system (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims. Having thus described the details and particularity required by the patent laws, what is claimed and desired protected by Letters Patent is set forth in the appended claims.

## Claims

1. An image guidance system (100), comprising:
a workstation (102) comprising a processor (104) and a memory (110) coupled to the processor;
an imaging system (124) coupled to the processor, and comprising an ultrasound imaging probe, the imaging system configured to generate images (114) based on use of the ultrasound imaging probe,
the imaging system further including a display (126) to permit user selection of areas of interest in the images,
the images and the user-selected areas of interest being referenced to an imaging coordinate system
the memory including a computation engine (116) configured to receive tracking data describing real-time spatial tracking of the position and pose of at least one spatially tracked device and of the ultrasound imaging probe in the tracking coordinate system,
the computation engine further being configured to combine the images (114), the areas of interest, and real time spatially tracked data of the at least one spatially tracked device by registering the imaging coordinate system and the tracking coordinate system into a global coordinate system, in order to provide measurement and/or location information, the registering comprising mapping the images in the imaging coordinate system to the tracking coordinate system, or vice versa, using a mathematical transform,
wherein the imaging system and computation engine are coupled by a bidirectional communication channel (122) configured to permit transmission of the images and the areas of interest to the computation engine and transmission of the measurement and/or location information to the imaging system.

2. The image guidance system as recited in claim 1, further comprising one or more objects (134, 138) visible in the images generated by the ultrasound imaging system (124), wherein the one or more objects (134, 138) comprises the at least one spatially tracked device.

3. The image guidance system as recited in claim 2, wherein the computation engine is further configured to construct one or more two-dimensional planes (302, 304), each plane showing an intersection of two or more of: the one or more objects and the areas of interest.

4. The image guidance system as recited in claim 2, wherein the one or more objects include a sensor (138), and wherein the computation engine (116) is further configured to receive real time data measurements from the sensor.

5. The image guidance system as recited in claim 2, wherein the imaging system is further configured to represent the one or more objects as a virtual object on the display.

6. The image guidance system as recited in claim 1, wherein the display of the imaging system is configured to permit the user to select areas of interest including at least one of: one or more critical structures (204, 310) and one or more anatomical sites.

7. The image guidance system as recited in claim 4, wherein the sensor is an untracked sensor, and wherein the display of the imaging system is configured to permit the user to select areas of interest including one or more locations representing the untracked sensor.

8. The image guidance system as recited in claim 4, wherein the sensor is a tracked sensor, and wherein the position of the sensor is spatially tracked in the tracking coordinate system.

9. The image guidance system as recited in claim 4, wherein the computation engine is further configured to process data of the sensor before transmission to the imaging system.

10. The image guidance system as recited in claim 1, wherein the computation engine is further configured to construct a spatial bounding box (502) for a target area.

11. The image guidance system as recited in claim 4, wherein the display is further configured to visualize data (404) of the sensor selectively based on the user selection and/or automatically based on a presence of a spatial bounding box and/or the areas of interest.

12. The image guidance system as recited in claim 2, wherein the computation engine is further configured to generate a notification based on at least one of: a distance between the one or more objects and the areas of interest, and data of a sensor.

13. The system as recited in claim 2, wherein the one or more objects include at least one of an introducer, a needle, a catheter, and a guidewire.

14. A method for image guidance, comprising:
generating (602) images of a non-biological, mechanical system using an imaging system comprising an ultrasound imaging probe, wherein said images comprise one or more objects visible in said images, at least one of said one or more objects being a spatially tracked device referenced to a tracking coordinate system;
selecting (604) areas of interest in the images using a display of the imaging system, the images and the user-selected areas of interest being referenced to an imaging coordinate system;
receiving tracking data describing real-time spatial tracking of the position and pose of the spatially tracked device and the ultrasound imaging probe in the tracking coordinate system;
combining (606) the images, the areas of interest, and real time spatially tracked data by registering the imaging coordinate system and the tracking coordinate system into a global coordinate system in order to provide measurement and/or location information, the registering comprising mapping the images in the imaging coordinate system to the tracking coordinate system, or vice versa, using a mathematical transform; and
transmitting (616) the images and the areas of interest to a computation engine and the measurement and/or location information to the imaging system using a bidirectional communication channel coupling the imaging system and the computation engine.

15. The method as recited in claim 14, wherein the one or more objects include a sensor, and the method further comprising visualizing data of the sensor selectively based on one of the selecting and/or automatically based on a presence of a spatial bounding box and/or the areas of interest.

16. The method as recited in claim 14, further comprising generating (614) a notification based on at least one of a distance between the one or more objects and the areas of interest, and data of a sensor.

## Patentansprüche

1. Bildführungssystem (100), umfassend:
eine Arbeitsstation (102), umfassend einen Prozessor (104) und einen Speicher (110), der mit dem Prozessor gekuppelt ist;
ein Bildgebungssystem (124), das mit dem Prozessor gekuppelt ist und eine Ultraschallbildgebungssonde umfasst, wobei das Bildgebungssystem ausgelegt ist, um Bilder (114) basierend auf der Nutzung der Ultraschallbildgebungssonde zu generieren,
wobei das Bildgebungssystem zudem eine Anzeige (126) einschließt, um dem Anwender die Auswahl von Interessensbereichen in den Bildern zu ermöglichen,
wobei die Bilder und die vom Anwender ausgewählten Interessensbereiche in einem Koordinatensystem referenziert sind,
wobei der Speicher ein Rechenwerk (116) einschließt, das ausgelegt ist, um Verfolgungsdaten zu empfangen, die die räumliche Echtzeitverfolgung der Position und Lage von mindestens einer räumlich verfolgten Vorrichtung und der Ultraschallbildgebungssonde im Verfolgungskoordinatensystem beschreiben,
wobei das Rechenwerk zudem ausgelegt ist, um die Bilder (114), die Interessensbereiche und die in Echtzeit räumlich verfolgten Daten von mindestens einer räumlich verfolgten Vorrichtung zu kombinieren, indem das Bildgebungskoordinatensystem und das Verfolgungskoordinatensystem in einem globalen Koordinatensystem registriert werden, um Mess- und/oder Ortungsinformationen bereitzustellen, wobei das Registrieren das Mapping der Bilder im Bildgebungskoordinatensystem gegenüber dem Verfolgungskoordinatensystem oder umgekehrt umfasst, wobei eine mathematische Umrechnung genutzt wird,
wobei das Bildgebungssystem und das Rechenwerk mittels eines bidirektionalen Kommunikationskanals (122) gekuppelt sind, ausgelegt, um die Übertragung der Bilder und der Interessensbereiche an das Rechenwerk und die Übertragung der Mess- und/oder Ortungsinformationen an das Bildgebungssystem zu ermöglichen.

2. Bildführungssystem nach Anspruch 1, zudem umfassend ein oder mehrere Objekte (134, 138), die in den vom Ultraschallbildgebungssystem (124) generierten Bildern sichtbar sind, wobei das eine oder die mehreren Objekte (134, 138) die mindestens eine räumlich verfolgte Vorrichtung umfassen.

3. Bildführungssystem nach Anspruch 2, wobei das Rechenwerk zudem ausgelegt ist, um eine oder mehrere zweidimensionale Ebenen (302, 304) zu konstruieren, wobei eine jede Ebene eine Überschneidung von zwei oder mehr des einen oder der mehreren Objekte und der Interessensbereiche zeigt.

4. Bildführungssystem nach Anspruch 2, wobei das eine oder die mehreren Objekte zudem einen Sensor (138) einschließen und wobei das Rechenwerk (116) zudem ausgelegt ist, um Echtzeitdatenmessungen vom Sensor zu empfangen.

5. Bildführungssystem nach Anspruch 2, wobei das Bildgebungssystem zudem ausgelegt ist, um das eine oder die mehreren Objekte als ein virtuelles Objekt auf der Anzeige darzustellen.

6. Bildführungssystem nach Anspruch 1, wobei die Anzeige des Bildgebungssystems ausgelegt ist, um dem Anwender zu ermöglichen, Interessensbereiche auszuwählen, die mindestens eine oder mehrere kritische Strukturen (204, 310) und/oder eine oder mehrere anatomische Bereiche einschließen.

7. Bildführungssystem nach Anspruch 4, wobei der Sensor ein nicht verfolgter Sensor ist und wobei die Anzeige des Bildgebungssystems ausgelegt ist, um den Anwender zu ermöglichen, Interessensbereiche auszuwählen, die eine oder mehrere Orte einschließen, die den nicht verfolgten Sensor darstellen.

8. Bildführungssystem nach Anspruch 4, wobei der Sensor ein verfolgter Sensor ist und wobei die Position des Sensors räumlich im Verfolgungskoordinatensystem verfolgt wird.

9. Bildführungssystem nach Anspruch 4, wobei das Rechenwerk zudem ausgelegt ist, um Daten des Sensors vor der Übertragung ans Bildgebungssystem zu verarbeiten.

10. Bildführungssystem nach Anspruch 1, wobei das Rechenwerk zudem ausgelegt ist, um einen räumlichen quaderförmigen Hüllkörper (502) für einen Zielbereich zu konstruieren.

11. Bildführungssystem nach Anspruch 4, wobei die Anzeige zudem ausgelegt ist, um Daten (404) des Sensors anzuzeigen, die selektiv auf der Auswahl des Anwenders basieren und/oder automatisch auf einer Anwesenheit eines räumlichen quaderförmigen Hüllkörpers und/oder der Interessensbereiche basieren.

12. Bildführungssystem nach Anspruch 2, wobei das Rechenwerk zudem ausgelegt ist, um eine Benachrichtigung zu generieren, basierend auf mindestens entweder einem Abstand zwischen dem einen oder mehreren Objekten und den Interessensbereichen und/oder Daten eines Sensors.

13. System nach Anspruch 2, wobei das eine oder die mehreren Objekte mindestens entweder einen Inserter, eine Nadel, einen Katheter und/oder einen Führungsdraht einschließen.

14. Bildführungsverfahren, umfassend:
das Generieren (602) von Bildern eines nicht biologischen mechanischen Systems unter Nutzung eines Bildgebungssystems, umfassend eine Ultraschallbildgebungssonde, wobei die Bilder ein oder mehrere Objekte umfassen, die auf den Bildern sichtbar sind, wobei mindestens eins des einen oder der mehreren Objekte eine räumlich verfolgte Vorrichtung ist, die in einem Verfolgungskoordinatensystem referenziert ist;
das Auswählen (604) von Interessensbereichen in den Bildern unter Nutzung einer Anzeige des Bildgebungssystems, wobei die Bilder und die vom Anwender ausgewählten Interessensbereiche in einem Koordinatensystem referenziert sind;
das Empfangen von Verfolgungsdaten, die die räumliche Echtzeitverfolgung der Position und Lage der räumlich verfolgten Vorrichtung und der Ultraschallbildgebungssonde im Verfolgungskoordinatensystem beschreiben;
das Kombinieren (606) der Bilder, der Interessensbereiche und der in Echtzeit räumlich verfolgten Daten, indem das Bildgebungskoordinatensystem und das Verfolgungskoordinatensystem in einem globalen Koordinatensystem registriert werden, um Mess- und/oder Ortungsinformationen bereitzustellen, wobei das Registrieren das Mapping der Bilder im Bildgebungskoordinatensystem gegenüber dem Verfolgungskoordinatensystem oder umgekehrt umfasst, wobei eine mathematische Umrechnung genutzt wird, und
das Übertragen (616) der Bilder und der Interessensbereiche an ein Rechenwerk und der Mess- und/oder Ortungsinformationen an das Bildgebungssystem unter Nutzung eines bidirektionalen Kommunikationskanals, indem das Bildgebungssystem und das Rechenwerk gekuppelt werden.

15. Verfahren nach Anspruch 14, wobei das eine oder die mehreren Objekte einen Sensor umfassen und das Verfahren zudem das Anzeigen von Daten des Sensors einschließt, die selektiv entweder auf dem Auswählen und/oder automatisch auf einer Anwesenheit eines räumlichen quaderförmigen Hüllkörpers und/oder der Interessensbereiche basieren.

16. Verfahren nach Anspruch 14, zudem umfassend das Generieren (614) einer Benachrichtigung, basierend auf mindestens entweder einem Abstand zwischen dem einen oder den mehreren Objekten und den Interessensbereichen und/oder Daten eines Sensors.

## Revendications

1. Système de guidage d'images (100), comprenant :
un poste informatique (102) comprenant un processeur (104) et une mémoire (110) couplée au processeur ;
un système d'imagerie (124) couplé au processeur, et comprenant une sonde d'imagerie ultrasonore, le système d'imagerie étant configuré pour générer des images (114) basées sur l'utilisation de la sonde d'imagerie ultrasonore,
le système d'imagerie incluant de plus un écran (126) pour permettre à l'utilisateur de sélectionner des zones d'intérêt dans les images,
les images et les zones d'intérêt sélectionnées par l'utilisateur étant référencées dans un système de coordonnées d'imagerie,
la mémoire incluant un moteur de calcul (116) configuré pour recevoir des données de suivi décrivant le suivi dans l'espace en temps réel de la position et de la pose d'au moins un dispositif suivi dans l'espace et de la sonde d'imagerie ultrasonore dans le système de coordonnées de suivi,
le moteur de calcul étant de plus configuré pour combiner les images (114), les zones d'intérêt et les données suivies dans l'espace en temps réel de l'au moins un dispositif suivi dans l'espace en enregistrant le système de coordonnées d'imagerie et le système de coordonnées de suivi dans un système de coordonnées global, afin de fournir des informations de mesure et/ou de localisation, l'enregistrement comprenant le mappage des images dans le système de coordonnées d'imagerie au système de coordonnées de suivi, ou vice versa, en utilisant une transformée mathématique,
dans lequel le système d'imagerie et le moteur de calcul sont couplés par un canal de communication (122) bidirectionnel configuré pour permettre la transmission des images et des zones d'intérêt au moteur de calcul et la transmission des informations de mesure et/ou de localisation au système d'imagerie.

2. Système de guidage d'images selon la revendication 1, comprenant de plus un ou plusieurs objets (134, 138) visibles dans les images générées par le système d'imagerie (124) par ultrasons, dans lequel un ou plusieurs objets (134, 138) comprennent l'au moins un dispositif suivi dans l'espace.

3. Système de guidage d'images selon la revendication 2, dans lequel le moteur de calcul est de plus configuré pour construire un ou plusieurs plans bidimensionnels (302, 304), chaque plan indiquant une intersection de deux ou plusieurs des éléments suivants : un ou plusieurs objets et les zones d'intérêt.

4. Système de guidage d'images selon la revendication 2, dans lequel un ou plusieurs objets incluent un capteur (138), et dans lequel le moteur de calcul (116) est de plus configuré pour recevoir des mesures de données en temps réel du capteur.

5. Système de guidage d'images selon la revendication 2, dans lequel le système d'imagerie est de plus configuré pour représenter un ou plusieurs objets comme un objet virtuel sur l'écran.

6. Système de guidage d'images selon la revendication 1, dans lequel l'affichage du système d'imagerie est configuré pour permettre à l'utilisateur de sélectionner des zones d'intérêt incluant au moins une des structures suivantes : une ou plusieurs structures critiques (204, 310) et un ou plusieurs sites anatomiques.

7. Système de guidage d'images selon la revendication 4, dans lequel le capteur est un capteur non suivi, et dans lequel l'affichage du système d'imagerie est configuré pour permettre à l'utilisateur de sélectionner des zones d'intérêt incluant un ou plusieurs emplacements représentant le capteur non suivi.

8. Système de guidage d'images selon la revendication 4, dans lequel le capteur est un capteur suivi, et dans lequel la position du capteur est suivi e dans l'espace dans le système de coordonnées de suivi.

9. Système de guidage d'images selon la revendication 4, dans lequel le moteur de calcul est de plus configuré pour traiter les données du capteur avant transmission au système d'imagerie.

10. Système de guidage d'images selon la revendication 1, dans lequel le moteur de calcul est de plus configuré pour construire un rectangle englobant de l'espace (502) pour une zone cible.

11. Système de guidage d'images selon la revendication 4, dans lequel l'affichage est de plus configuré pour visualiser les données (404) du capteur sélectivement sur la base de la sélection de l'utilisateur et/ou automatiquement sur la base d'une présence d'un rectangle englobant de l'espace et/ou des zones d'intérêt.

12. Système de guidage d'images selon la revendication 2, dans lequel le moteur de calcul est de plus configuré pour générer une notification basée sur au moins l'un des éléments suivants : une distance entre un ou plusieurs objets et les zones d'intérêt, et les données d'un capteur.

13. Système selon la revendication 2, dans lequel un ou plusieurs objets incluent au moins un des éléments suivants : un intubateur, une aiguille, un cathéter et un fil guide.

14. Procédé de guidage d'images, comprenant :
générer (602) des images d'un système mécanique non biologique en utilisant un système d'imagerie comprenant une sonde d'imagerie ultrasonore, dans lequel lesdites images comprennent un ou plusieurs objets visibles dans lesdites images, au moins un desdits un ou plusieurs objets étant un dispositif suivi dans l'espace référencé à un système de coordonnées de suivi ;
sélectionner (604) des zones d'intérêt dans les images en utilisant un affichage du système d'imagerie, les images et les zones d'intérêt sélectionnées par l'utilisateur étant référencées à un système de coordonnées d'imagerie ;
recevoir des données de suivi décrivant le suivi dans l'espace en temps réel de la position et de la pose du dispositif suivi dans l'espace et de la sonde d'imagerie ultrasonore dans le système de coordonnées de suivi ;
combiner (606) les images, les zones d'intérêt et les données de suivi dans l'espace en temps réel en enregistrant le système de coordonnées d'imagerie et le système de coordonnées de suivi dans un système de coordonnées global afin de fournir des informations de mesure et/ou de localisation, l'enregistrement comprenant le mappage des images dans le système de coordonnées d'imagerie au système de coordonnées de suivi, ou vice versa, en utilisant une transformée mathématique ; et
transmettre (616) les images et les zones d'intérêt à un moteur de calcul et les informations de mesure et/ou de localisation au système d'imagerie en utilisant un canal de communication bidirectionnel couplant le système d'imagerie et le moteur de calcul.

15. Procédé selon la revendication 14, dans lequel un ou plusieurs objets incluent un capteur, et le procédé comprenant de plus la visualisation des données du capteur sélectivement sur la base de l'une des sélections et/ou automatiquement sur la base d'une présence d'un rectangle englobant de l'espace et/ou des zones d'intérêt.

16. Procédé selon la revendication 14, comprenant de plus la génération (614) d'une notification basée sur au moins l'un des éléments suivants : une distance entre un ou plusieurs objets et les zones d'intérêt, et les données d'un capteur.
